(19) Europäisches Patentamt
European Patent Office
Office européen des brevets

(11) EP 4 331 624 A1

(12) EUROPEAN PATENT APPLICATION
published in accordance with Art. 153(4) EPC

(43) Date of publication:
**06.03.2024 Bulletin 2024/10**

(21) Application number: **21939473.1**

(22) Date of filing: **14.10.2021**

(51) International Patent Classification (IPC):
***A61K 51/04*** *(2006.01)* ***A61K 31/69*** *(2006.01)*
***G01N 33/60*** *(2006.01)* ***G01N 33/574*** *(2006.01)*
***C07D 401/12*** *(2006.01)* ***C07F 13/00*** *(2006.01)*
***C07F 5/02*** *(2006.01)* ***A61P 35/00*** *(2006.01)*

(52) Cooperative Patent Classification (CPC):
**A61K 31/69; A61K 51/04; A61P 35/00; C07D 401/12; C07F 5/02; C07F 13/00; G01N 33/574; G01N 33/60**

(86) International application number:
**PCT/MX2021/050055**

(87) International publication number:
**WO 2022/231410 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2021 MX 2021005089**

(71) Applicant: **Instituto Nacional de Investigaciones Nucleares**
**Ocoyoacac, Estado de México 52750 (MX)**

(72) Inventors:
- **LUNA-GUTIÉRREZ, Myrna Alejandra**
  **OCOYOACAC, 52750 (MX)**
- **SANTOS-CUEVAS, Clara Leticia**
  **OCOYOACAC, 52750 (MX)**
- **JIMÉNEZ-MANCILLA, Nallely Patricia**
  **OCOYOACAC, 52750 (MX)**
- **AZORIN-VEGA, Erika Patricia**
  **OCOYOACAC, 52750 (MX)**
- **FERRO-FLORES, Guillermina**
  **OCOYOACAC, 52750 (MX)**
- **OCAMPO-GARCÍA, Blanca Elí**
  **OCOYOACAC, 52750 (MX)**

(74) Representative: **De Clercq & Partners**
**Edgard Gevaertdreef 10a**
**9830 Sint-Martens-Latem (BE)**

(54) **RADIOPHARMACEUTICALS BASED ON ((R)-1-((6-HYDRAZINYLNICOTINOYL)-D-ALANYL)PYRROLIDIN-2-YL)BORONIC ACID (HYNIC-IFAP) FOR DETECTING THE OVEREXPRESSION OF FIBROBLAST ACTIVATION PROTEIN**

(57) This invention relates to new fibroblast activation protein (iFAP) inhibitory radiopharmaceuticals based on the molecule ((R)-1-((6-hydrazinylnicotinoyl)-D-alanyl)pyrrolidin-2-yl)boronic acid (HYNIC-iFAP), where the hydrazine nitrogens of HYNIC act as favorable chemical groups for the interaction of the HYNIC-iFAP molecule with phenylalanine (Phe-350 and Phe-351), glutamic acid (Glu-203 and Glu-204) and with serine (Ser-624) in the active center of fibroblast activation protein (FAP), coupled with the conventional use of HYNIC as a chelating agent for the radiometal $^{99m}$Tc, where ethylenediamine diacetic acid (EDDA) is used to complete the coordination sphere of the radiometal. The new radiopharmaceutical from $^{99m}$Tc-EDDA/HYNIC-iFAP ($^{99m}$Tc-HYNIC-iFAP) detects, with high affinity *in vivo,* FAP expressed in the microenvironment of malignant tumors of epithelial origin using nuclear medicine SPECT molecular imaging techniques.

The object of this invention is to provide a new SPECT-specific radiopharmaceutical with high sensitivity for the detection of FAP protein expression in the tumor microenvironment, based on boronPro-type inhibitors (molecular target radiopharmaceutical).

EP 4 331 624 A1

## Description

TECHNICAL FIELD OF THE INVENTION

The present invention relates to new radiopharmaceuticals based on the fibroblast activation protein (iFAP) inhibitor ((R)-1-((6-hydrazinylnicotinoyl)-D-alanyl)pyrrolidin-2-yl)boronic acid (HYNIC-iFAP), where the hydrazine nitrogens of HYNIC act as favorable chemical groups for the interaction of the HYNIC-iFAP molecule with phenylalanine (Phe-350 and Phe-351), glutamic acid (Glu-203 and Glu-204) and serine (Ser-624), the essential active center of fibroblast activation protein (FAP), coupled with the conventional use of HYNIC as a chelating agent for the radiometal $^{99m}$Tc. In particular, the new radiopharmaceutical HYNIC-iFAP labeled with $^{99m}$Tc, detects with high affinity *in vivo,* the FAP expressed in the tumor microenvironment by SPECT molecular imaging techniques in nuclear medicine.

BACKGROUND

Fibroblast activation protein (FAP) is a type II serine protease that cleaves peptides located downstream of proline residues with dipeptidyl-peptidase and endopeptidase activities. FAP is highly expressed on the cell surface of the activated stromal fibroblast present in most human epithelial tumors, but not in normal fibroblasts. Cancer-associated fibroblasts contribute up to 90% of the macroscopic tumor mass *[*Hamson et al. Understanding fibroblast activation protein (FAP): substrates, activities, expression and targeting for cancer therapy. Proteomics Clin. Appl., 2014, 8, 454-463].

Specific FAP inhibitors were first developed as potential anticancer drugs [Aertgeerts et al. Structural and kinetic analysis of the substrate specificity of human fibroblast activation protein a. J. Biol. Chem., 2005, 280, 19441-19444*;* Edosada et al. Selective inhibition of fibroblast activation protein protease based on dipeptide substrate specificity. J. Biol. Chem. 2006, 281, 7437-7444; Tran et al. Synthesis and structure-activity relationship of N-acyl-Gly-, N-acyl-Sar- and N-blocked-boroPro inhibitors of FAP, DPP4, and POP. Bioorg. Med. Chem. Lett., 17, 2007, 1438-1442*].* Of these, the most relevant are two groups of highly selective compounds, one based on a quinoline-cyanopyrrolidin structure and the other based on pyrrolidin-boronic acid or also known as proline-boronic acid (boronPro). Regarding the first group, Jansen et al. initially reported the synthesis of 39 new FAP inhibitors to explore the structure-activity relationship of the 4-quinolinoyl-Glycyanopyrrolidin scaffold *[*Jansen et al. Extended structure activity relationship and pharmacokinetic investigation of (4-quinolinoyl)-glycyl-2-cyanopyrrolidin inhibitors of fibroblast activation protein J. Med. Chem., 2014, 57, 3053-3074*].* The authors reported that FAP imposes stringent structural requirements in order to form covalent bonds between the enzyme and the nitrile group present in these molecules *[*Jansen et al. Selective inhibitors of fibroblast activation protein (FAP) with a (4-quinolinoyl)-glycyl-2-cyanopyrrolidin scaffold. ACS Med. Chem. Lett., 2013, 4, 491-496]. They also found that N-pyridines give rise to FAP inhibitors of high selectivity towards other post-proline cleavage enzymes, such as dipeptidyl-peptidases (DPP) and prolyl-oligopeptidase (PREP). Although the quinolinoyl fragment confers the molecule a higher affinity for FAP, when this residue was replaced by another azaheteroaromatic substituent, the affinity for FAP decreased drastically. Furthermore, the addition of fluorine or difluoro to the cyanopyrrolidin ring fragment was found to improve the affinity and selectivity for FAP. When the authors replaced the glycine residue with some other amino acids, the affinity for PREP increased and the potency of FAP decreased significantly. As a result of this study, the authors identified the N-(4-quinolinoyl)-glycyl-(2-cyanopyrrolidin) scaffold as the best inhibitor of FAP. Pharmacokinetic studies in mice of the selected FAP inhibitor showed high bioavailability after oral administration, with a short plasma half-life and long-lasting *in vivo* FAP inhibition.

At the same time, Poplawsky et al. designed and characterized more than 20 proline-boronic-based inhibitors for FAP and PREP *[*Poplawski et al. Identification of selective and potent inhibitors of fibroblast activation protein and prolyl oligopeptidase. J. Med. Chem. 2013, 56, 3467-3477]. The authors reported that the affinity for FAP could be increased by using a protonated pyridinic nitrogen atom, because it forms a hydrogen bond with the carbonyl oxygen of glutamic acid (Glu-204) present in FAP, but absent in PREP. The authors also found that the endopeptidase activity of FAP has extremely stringent requirements, accepting only small amino acids such as glycine or D-alanine. Although D-alanine is preferred because the inhibitor retains FAP inhibitory potency in the nanomolar range and provides 360-fold selectivity for FAP over PREP. Based on these results, Poplawski et al. established that N-(pyridin-4-carbonyl)-D-Ala-boroPro was the best fibroblast activation protein inhibitor found.

Despite the large number of synthesized and characterized FAP inhibitors, only a limited number of them have been radiolabeled for medical purposes. In 2015, the first radiolabeled boronic acid-based FAP inhibitor with iodine-125 ($^{125}$ I-MIP-1232) was reported for atherosclerotic plaque imaging, although it is important to clarify that I-125 is not a useful radionuclide for single photon emission computed tomography (SPECT) or positron emission tomography (PET) imaging *[*Meletta et al. Evaluation of the radiolabeled boronic acid based FAP inhibitor MIP-1232 for atherosclerotic plaque imaging. Molecules, 2015, 20, 2081-2099*].*

In 2018, Lindner and Loktev reported the conjugation of 2,2',2",2"'-(1,4,7,10-tetraazacyclododecane-1,4,7,10-tetrayl)tetraacetic acid (DOTA) to the 4-quinolinoyl-Gly- cyanopyrrolidin scaffold in order to label it with suitable radionuclides for diagnostic imaging or therapeutic purposes *[*Lindner et al. Development of quinoline-based theranostic ligands for the targeting of fibroblast activation protein. J. Nucl. Med., 2018, 59, 1415-1422*;* Loktev et al. A tumor-imaging method targeting cancer-associated fibroblasts. J. Nucl. Med., 2018, 59, 1423-1429*]*. The authors developed fifteen derivatives using different positions to conjugate DOTA to the 4-quinolinoyl-Gly-cyanopyrrolidin scaffold in order to improve tumor uptake and retention time of FAP inhibitor (FAPI) derivatives. The 15 different FAPIs were radiolabeled with $^{177}$Lu, $^{90}$Y or $^{68}$Ga. Of all the radiolabeled and reported FAPI complexes, $^{68}$Ga-FAPI-02 and $^{68}$Ga-FAPI-04 proved to be the most suitable agents for diagnostic applications. Subsequently, the same authors reported other derivatives of the same framework numbered consecutively from FAPI-21 to FAPI-55 *[*Loktev et al. Development of Fibroblast Activation Protein-Targeted Radiotracers with Improved Tumor Retention. J. Nucl. Med., 2019, 60,1421-1429*]*. Substitution of DOTA with 1,4,7-triazacyclononane-N,N',N"-triacetic acid (NOTA) gave rise to the derivative FAPI-74, which can be labeled with $^{68}$Ga and with $^{18}$F ($^{18}$*F-AIF,* in the presence of $AlCl_3$ ) *[*Giesel et al. FAPI-74 PET/CT Using Either 18F-AIF or Cold-Kit 68Ga Labeling: Biodistribution, Radiation Dosimetry, and Tumor Delineation in Lung Cancer Patients. J. Nucl. Med., 2021, 62, 201-207*]*. In particular, FAPI-35 was developed for labeling with $^{99m}$Tc and possibly $^{188}$Re *[*Lindner et al. Design and Development of 99mTc-Labeled FAPI Tracers for SPECT Imaging and 188Re Therapy. J. Nucl. Med., 2020, 61, 1507-1513*]*.

The first FAP inhibitors used in humans for PET scans were $^{68}$Ga-FAPI-02 and $^{68}$Ga-FAPI-04 *[*Giesel etal. 68Ga-FAPI PET/CT: biodistribution and preliminary dosimetry estimate of 2 DOTA-containing FAP-targeting agents in patients with various cancers. J. Nucl. Med., 2019, 60, 386-392; Kratochwil et al. 68Ga-FAPI PET/CT: Tracer Uptake in 28 Different Kinds of Cancer J. Nucl. Med., 2019, 60, 801-805*]*.

Interestingly, the $^{64}$Cu-/$^{225}$Ac-FAPI-04 theranostic couple, which demonstrated its utility in the treatment of pancreatic cancer overexpressing FAP in murine models. Therapy targeting fibroblast activation protein is effective in cancer treatment and could contribute to the establishment of new treatment strategies [Watabe et al. Theranostics Targeting Fibroblast Activation Protein in the Tumor Stroma: 64Cu- and 22 Ac-Labeled FAPI-04 in Pancreatic Cancer Xenograft Mouse Models. J. Nucl. Med., 2020, 61, 563-569].

However, prior to any radiotherapeutic treatment, the uptake of the radiopharmaceutical in the tumors or their metastases must be evaluated by nuclear imaging in order to confirm whether or not the treatment will be useful for the patient, as well as to determine the necessary activity to administer in order to deliver the ablative radiation dose to the tumors, i.e. personalized medicine is practiced. For this, it is necessary to use FAP inhibitor diagnostic radiopharmaceuticals in order to obtain molecular images by PET or SPECT. Of these two techniques, PET is the one with the highest spatial resolution and sensitivity, so that, as described above, the diagnostic FAP inhibitor radiopharmaceuticals so far developed and applied in clinical studies, use $^{68}$Ga and $^{18}$F bound to 4-quinolinoyl-Gly-cyanopyrrolidin derivatives, which are radiopharmaceuticals for PET, and only one study has used a derivative of $^{99m}$Tc also bound to quinoline-cyanopyrrolidin (see table below). Nevertheless, nationally and internationally, SPECT studies represent more than 70% of the total in nuclear medicine due to their lower cost and greater availability of equipment and radionuclides, since it is not necessary to have a cyclotron in or near hospitals. For SPECT imaging, the most commonly used radionuclide is $^{99m}$Tc, and there is no publication dedicated to a study on radiopharmaceuticals for FAP imaging based on pyrrolidinboronic acid derivatives or boron-Pro.

Table 1: Chemical structure of radiopharmaceuticals of $^{68}$Ga,$^{18}$F and $^{99m}$Tc FAP inhibitors so far developed and used in clinical studies by PET and SPECT techniques.

| CHEMICAL STRUCTURE | NAME AND USE |
| --- | --- |
| 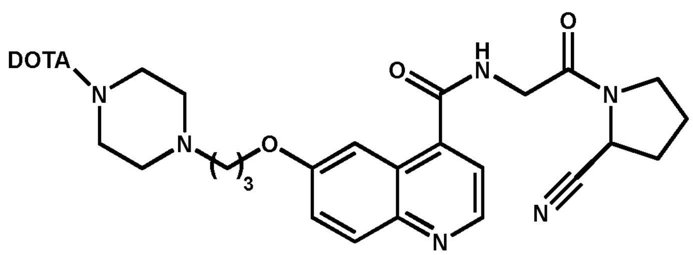 | FAPI-02 Radiolabeled with $^{68}$Ga PET imaging |

(continued)

| CHEMICAL STRUCTURE | NAME AND USE |
|---|---|
| 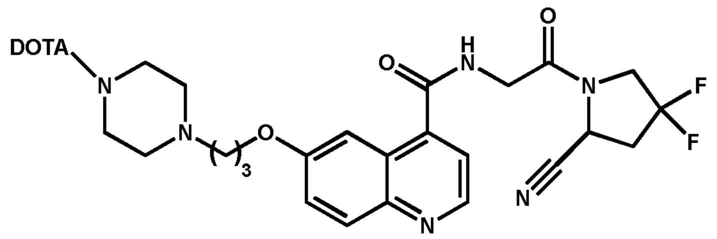 | FAPI-04 Radiolabeled with $^{68}Ga$ PET imaging |
| 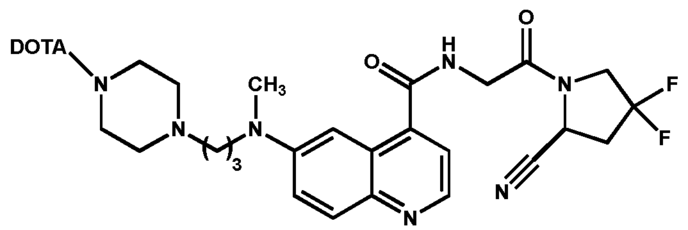 | FAPI-46 Radiolabeled with $^{68}Ga$ PET imaging |
| 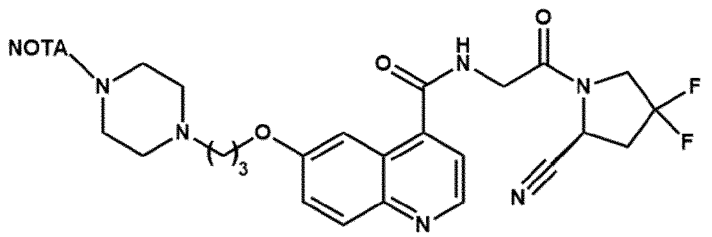 | FAPI-74 Radiolabeled with $^{68}Ga$ and $^{18}F$ PET imaging |
| 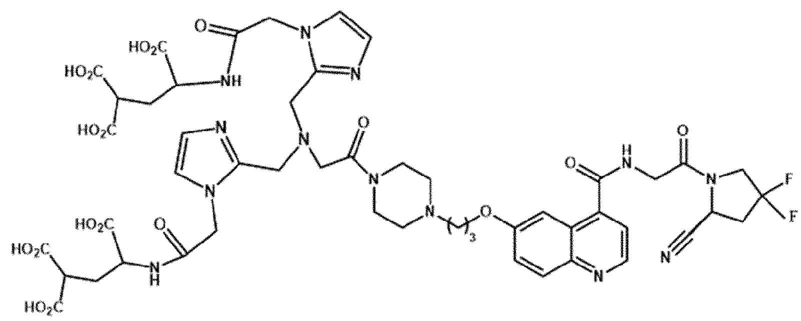 | FAPI-34 Radiolabeled with $^{99m}Tc$ SPECT imaging |

DETAILED DESCRIPTION OF THE INVENTION

Novel fibroblast activation protein (iFAP) inhibitory radiopharmaceuticals based on the ((R)-1-((6-hydrazinylnicotinoyl)-D-alanyl)pyrrolidin-2-yl)boronic acid (HYNIC-iFAP) molecule are presented for patent purposes, where the hydrazine nitrogens of HYNIC act as favorable chemical groups for the interaction of the HYNIC-iFAP molecule with phenylalanine (Phe-350 and Phe-351), glutamic acid (Glu-203 and Glu-204) and with serine (Ser-624) in the active center of fibroblast activation protein (FAP), coupled with the conventional use of HYNIC as a chelating agent for the radiometal $^{99m}Tc$, where ethylenediamine diacetic acid (EDDA) is used to complete the coordination sphere of the radiometal. The new radiopharmaceutical from $^{99m}Tc$-EDDA/HYNIC-iFAP ($^{99m}Tc$-HYNIC-iFAP) detects, with high affinity *in vivo,* FAP expressed in the microenvironment of malignant tumors of epithelial origin using nuclear medicine SPECT molecular imaging techniques. **Figure 1 shows** schematically the structure of the $^{99m}Tc$ radiopharmaceutical to be patented based on the HYNIC-iFAP structure.

Based on a molecular docking study, the HYNIC-iFAP derivative [((R)-1-((6hydrazinylnicotinoyl-)-Dalanyl)-pyrrolidin-2-yl)boronic] was designed and synthesized, which was compared in affinity and inhibition constant (*Ki*) with two of the most representative structures of boronPro reported in the literature, N-acyl-Gly-boroPro *[*Tran et al. Synthesis and structure-activity relationship of N-acyl-Gly-, N-acyl-Sar- and N-blocked-boroPro inhibitors of FAP, DPP4, and POP. Bioorg. Med. Chem. Lett., 17, 2007, 1438-1442*]* and N-(pyridin-4-carbonyl)-D-Ala-boroPro *[*Poplawski et al. Identification

of selective and potent inhibitors of fibroblast activation protein and prolyl oligopeptidase. J. Med. Chem. 2013, 56, 3467-3477]. The affinity of another derivative of the HYNIC-iFAP molecule, where it was conjugated to S-2-(4-lsothiocyanatobenzyl)-DOTA (DOTA-Bz-NCS-HYN!C-iFAP), was also obtained in order to study whether the affinity of this derivative is suitable for potential labeling with other DOTA-like radiometals, such as $^{68}$Ga,$^{177}$Lu, $^{64}$Cu, $^{225}$Ac, etc. For this purpose, all structures were generated in ChemDraw (.cdx format) and the 3D structure was exported in .pdb format via Chem3D software. Using the molecular editor AVOGADRO 1.2.0, the molecular geometry was optimized using a universal force field (UFF), due to the presence of a boron atom in each structure, with a total of 10,000 steps. Subsequently, a second geometry optimization was performed using the semi-empirical Quantum Chemistry software MOPAC2016 with a PM7 level of theory exporting the resulting spatial configuration to .pdb format.

The crystal structure of the alpha subunit of fibroblast activating protein (FAP) was obtained from the RSCB Protein Data Bank database (PDB ID: 1Z68). In order to use it as a receptor macromolecule in molecular docking calculations, the molecule was edited in BIOVIA Discovery Studio 2021 to remove water molecules and residues manifested in X-ray diffraction, leaving only the main amino acid chain in the .pdb file. The A chain of the dimer represented in the model was also removed, leaving only the B chain. Because the three-dimensional model of the macromolecule involves a resolution of 2.60 Å, a homology modeling step was performed through the SWISS-MODEL online platform. The resulting structure was saved in pdb format for use as a receptor. Both the receptor and the HYNIC-iFAP-derived structures were prepared with the OPEN BABEL GUI 2006 library indicating the addition of missing hydrogens and their molecular optimization for physiological pH (pH=7.4), again generating the structures in .pdb format. The AutoDock Tools 1.5.6 software package was used to configure the receptor as a macromolecule and each of the ligands in separate files, exporting the files in .pdbqt extension. The search box was configured at the hydrophobic site S1 around the Ser-624 of the receptor as suggested by Poplawsky and coworkers in 2013, as a center the XYZ coordinates 18.948, 10.676, 28.989 respectively and a size of 90 on each axis of the box were set. Regarding the ligands it was necessary to modify the <<AD4_parameters.dat>> file to add the parameters for the Boron atom available at http://mgldev.scripps.edu/pipermail/autodock/2009-March/005439.html.

The execution of the protein-ligand molecular docking was performed with the AutoDock 4.2.6 package, previously calculating the necessary .map grids with the AutoGrid 4.2.6 tool. The log file with the molecular docking results was visualized in AutoDock Tools by exporting to .pdb format the complex with the best affinity score. Visualization and analysis of distances and interactions was performed by BIOVA Discovery Studio Visualizer 2021.

The inhibition constant (*Ki*) was calculated using the equation:

$$Ki = e^{[\frac{Affinity}{RT}]}$$

Where:

*Affinity.* Corresponds to the value obtained in the AutoDock 4.2.6 scoring function.
R: Corresponds to the value of the thermodynamic constant of ideal gases (0.00198179 kcal/mol K).
T: Corresponds to the temperature value in absolute scale 298.15 K that AutoDock 4.2.6 handles in the molecular coupling calculations.

The Ki value is obtained in Molar units (M). **Table** 2 presents the affinity scores, Ki inhibition constants, and distance to Ser624 for each ligand. As evidenced in **Table 2,** the HYNIC-iFAP molecule showed a 26-fold lower inhibition constant (Ki=0.536 nM) with respect to the N-(pyridin-4-carbonyl)-D-Ala-boroPro derivative (Ki=14.07), which means a higher inhibitory potency of HYNIC-iFAP by FAP.

TABLE 2. Affinity scores determined by molecular docking (AutoDock), as well as the inhibition constants (*Ki*), and interaction distance between the boron residue and Serine 624 of fibroblast activation protein (FAP) for each boroPro ligand.

| boroPro Ligand | Affinity (kcal/mol) | *Ki* (nM) | Distance Ser624-B (Å) |
|---|---|---|---|
| **N-acyl-Gly-boroPro** | | | |

(continued)

| boroPro Ligand | Affinity (kcal/mol) | *Ki* (nM) | Distance Ser624-B (Å) |
|---|---|---|---|
| 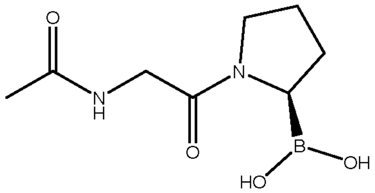 | -4.80 | 296.4 6 | 3.398 |
| **N-(pyridi ne-4-carbonyl)-D-Ala-boron Pro)** | | | |
| 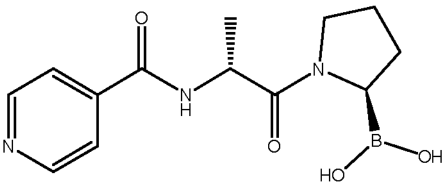 | -6.60 | 14.07 | 3.657 |
| **HYNIC-iFAP** | | | |
| 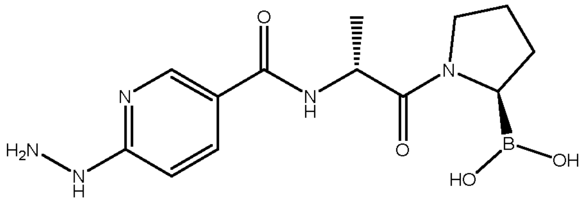 | -8.53 | 0.536 | 3.274 |
| **DOTA-Bz- NCS -HYNIC-iFAP** | | | |
| 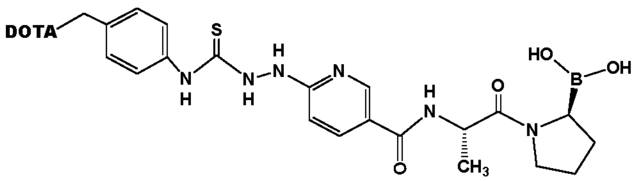 | -8.89 | 0.292 | 4.496 |

In agreement with the interaction map of HYNIC-iFAP and the amino acid residues of the FAP active center obtained in the molecular docking study, the increased affinity of HYNIC-iFAP, with respect to N-(pyridin-4-carbonyl)-D-Ala-boroPro, is due to the presence of the hydrazine nitrogens of HYNIC, which favor van der Waals-type interactions and hydrogen bonds of the HYNIC-iFAP molecule in its coupling to the active center of FAP, mainly with residues Glu-203, Glu-204, Phe-350 and Phe-351, as well as a closer proximity for interaction with Ser-624 (interaction map of amino acid residues of FAP with the HYNIC-iFAP ligand, obtained with the molecular docking methodology described above). The high affinity and FAP inhibitory potency of the DOTA-Bz-NCS-HYNIC-iFAP ligand indicates its potential to be used in the preparation of new diagnostic and therapeutic radiopharmaceuticals, as it could be a useful ligand to be labeled with radionuclides capable of being chelated by the DOTA macrocycle, such as $^{68}Ga$, $^{64}Cu$,$^{177}Lu$ and $^{225}Ac$.

<u>Method of preparation of the radiopharmaceutical of the invention</u>

For the synthesis of the HYNIC-iFAP molecule, Boc-pyrrolidin was initially dissolved in ethyl ether/TMEDA under a nitrogen atmosphere and at -40 °C. It was subsequently reacted with a solution of s-BuLi (in cyclohexane) at 5 °C. $B(OMe)_3$ was then added and the extraction purification process was carried out (first extraction with 2M NaOH, followed by acidification with 2M HCl, final extraction with EtOAc and evaporation of the solvent), obtaining Boc-pyrrolidin-boronic acid, to which pinanediol was added and the R diasteroisomer was obtained by HPLC separation (microporasil column). After Boc deprotection, D-alanine coupling was performed followed by succinimidyl-N-boc-HYNIC coupling using HATU/DIPEA. Finally, the compound was deprotected with TFA, purified by reverse phase HPLC and lyophilized. The final product was ((R)-1-((-6hydrazinylnicotinoyl-)-Dalanyl)pyrrolidin-2-yl)boronic acid (HYNIC-iFAP), which presented the expected mass spectrum: m/z 322 (calcd. 321) $[M+H]^+$ ; m/z 642 (calcd. 321) $2\times[M+H]^+$. $^1H$-NMR (300 MHz, DMSO),$\delta$ (ppm): 8.5-6.7 (s, 1H, $-CH^6$ -, arom. pyridine), 9.7 (s, 1H, *-NH-NH-),* 8.3 (s, 1H, $-CH_2$ -NHCO), 3.1-3.3 (m, 1H, $CH_2$ *CHB).*

Reversed-phase HPLC analysis of the freeze-dried white solid showed a chemical purity of the compound of 95 %.

HYNIC-iFAP (30 μg) was formulated as a lyophilized dosage form containing 10 mg EDDA, 20 mg tricine, 20 μg stannous chloride and 50 mg mannitol. Said formulation, when reconstituted with 1 mL of a solution of 0.2 M, pH 7 and 1 mL of sodium pertechnetate solution ($^{99m}TcO_4Na$), obtained *in situ* from a $^{99}Mo/^{99m}Tc$ generator, yields the compound to be patented $^{99m}$Tc-EDDA/HYNIC-iFAP ($^{99m}$Tc-HYNIC-iFAP) with a radiochemical purity greater than 98% as determined by reverse phase HPLC.

The radiopharmaceutical remains stable with a radiochemical purity greater than 95% after 24 h of labeling. *In vitro* stability tests in human serum show serum protein binding of 2.1 ± 0.3 % and high radiochemical stability (>95%). For the evaluation of the *in vitro* specificity of $^{99m}$Tc HYNIC-iFAP, we used tumor stroma (patient biopsy) from two different molecular subtypes of breast cancer: a luminal B, characterized by high expression of estrogen receptor (ER) and human epidermal growth factor receptor type 2 (HER2), and a triple negative (TNBC) due to null expression levels of ER, progesterone receptor (PR) and HER2. We chose these tumor stroma as in a previous study in different breast tumor phenotypes, TNBC tumors were shown to express the highest levels of FAP while luminal B tumors expressed the lowest levels of FAP [Park et al. Differential expression of cancer-associated fibroblast-related proteins according to molecular subtype and stromal histology in breast cancer Breast. Cancer Res. Treat., 2015, 149, 727-741]. The results showed an uptake of $^{99m}$Tc HYNIC-iFAP of 7.8 ± 1.2% of the radioactivity added to the stroma of TNBC tumors, which was significantly higher (P<0.05, t-student) than the radioactivity taken up by the stroma of luminal B tumors (2.3 ± 0.3% of the radioactivity added).

The compound showed no toxicity or adverse effects when administered at a dose of 40 mg/kg to laboratory balb-C mice. Micro-SPECT/CT imaging of $^{99m}$Tc-HYNIC-iFAP in athymic mice with induced triple-negative breast cancer tumors (MBCDF-T cells) showed an uptake in tumors of 9.2 ± 1.4% of the administered activity per gram of tissue (%ID/g) **(Figure 2)** with rapid elimination mainly via the kidney.

Biokinetic and dosimetry tests in healthy volunteers show rapid blood clearance with increased renal uptake and excretion, and an effective dose of 2.0 ± 0.5 mSv per 740 MBq administered. **Figure 3** shows a SPECT image of the radiopharmaceutical $^{99m}$Tc-HYNIC-iFAP obtained in a healthy volunteer at 1 h post-administration. **Figure 4** shows the PET and SPECT image of the same patient with triple negative breast cancer who was administered both $^{18}$F-FDG (PET, control radiopharmaceutical, gold standard for detection of tumor metabolism) and $^{99m}$Tc-HYNIC-iFAP (SPECT), showing that both radiopharmaceuticals detect with high sensitivity breast cancer tumors; **Figure 5** shows the PET and SPECT image of the same lung cancer patient (poorly differentiated lung adenocarcinoma with predominantly solid pattern) who was administered both $^{18}$F-FDG (PET, control radiopharmaceutical, gold standard for the detection of tumor metabolism) and $^{99m}$Tc-HYNIC-iFAP (SPECT), showing that both radiopharmaceuticals detect lung tumors with high sensitivity, although in the case of $^{99m}$Tc-HYNIC-iFAP, the high uptake is associated with the recognition of FAP expression in the tumor microenvironment. These images confirm and are the main evidence that, due to its FAP activity inhibition properties enhanced by the nitrogens present in HYNIC hydrazine, the $^{99m}$Tc-HYNIC-iFAP is able to detect tumor lesions in a specific manner.

In conclusion, the $^{99m}$Tc-HYNIC-iFAP is obtained with the following characteristics:

- Radiochemical purity greater than 98%.
- Ability of the radiopharmaceutical to detect *in vivo* and specifically to the microenvironment of tumors expressing fibroblast activation protein by nuclear medicine single photon emission computed tomography (SPECT).
- In addition to the molecular recognition of the boronPro residue, the radiopharmaceutical to be patented based on $^{99m}$Tc, has the ability to significantly capture and detect with high sensitivity the FAP-expressing tumor microenvironment, due to the increased affinity (low *Ki* value; *Ki=0.536)* conferred by the presence of the hydrazine nitrogens in the HYNIC molecule, which allows it to interact efficiently in docking with the active site of the FAP enzyme for detection by SPECT imaging.

## Claims

**1.** The radiopharmaceutical of the chemical formula $^{99m}$Tc-EDDA/HYNIC-iFAP ($^{99m}$Tc-HYNIC-iFAP), comprising the structure:

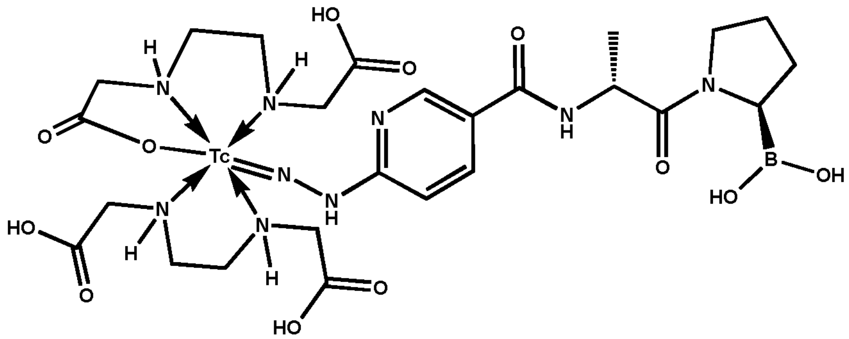

2. A radiopharmaceutical composition, **characterized in that** it comprises the radiopharmaceutical as claimed in claim 1.

3. The radiopharmaceutical claimed in claim 1 for use as a radiodiagnostic agent.

4. The precursor ligand of radiopharmaceuticals comprising the structure:

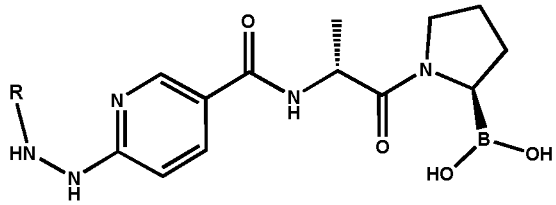

R=H (1)

R= 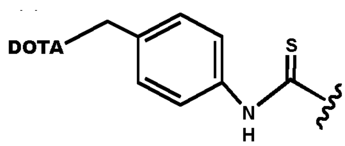 (2)

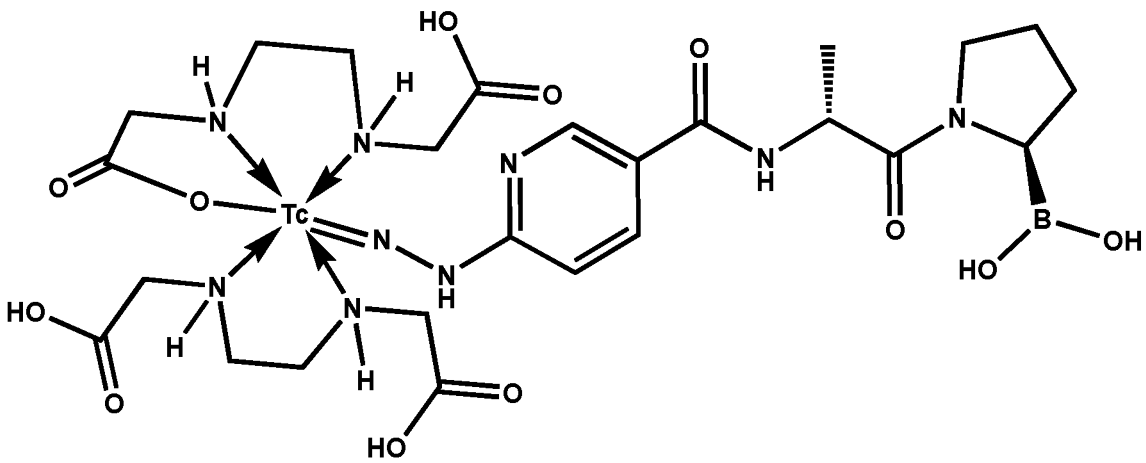

Figure 1

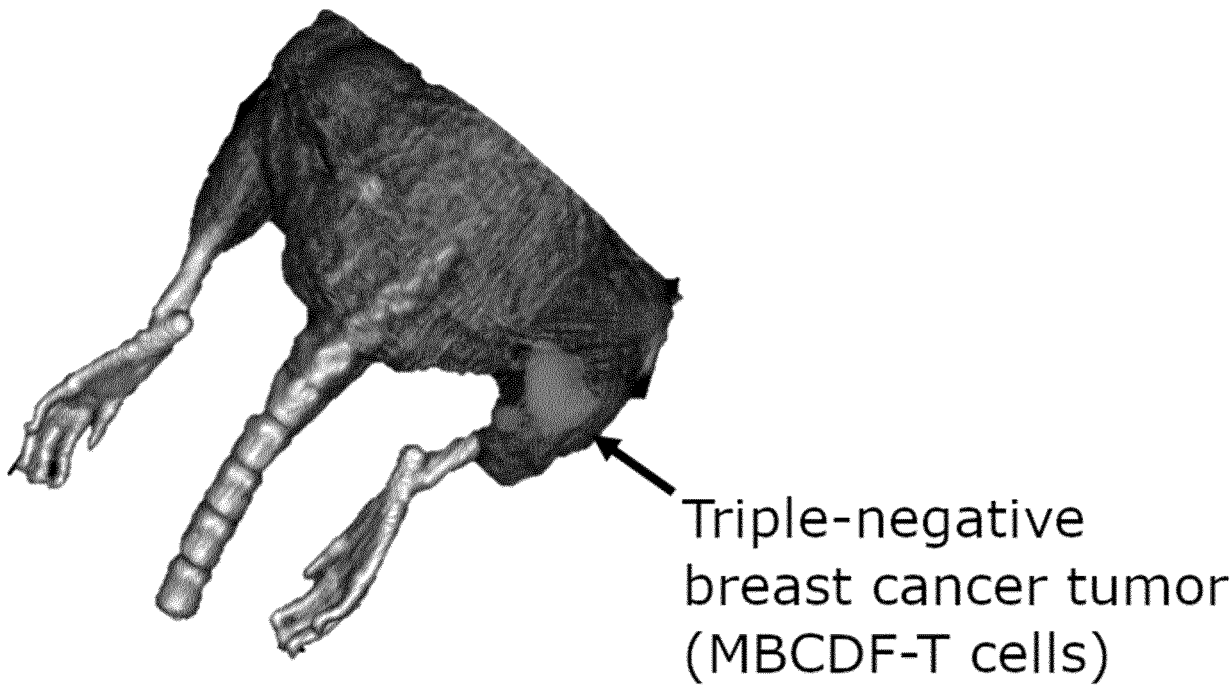


Figure 2

SPECT $^{99m}$Tc-HYNIC-iFAP

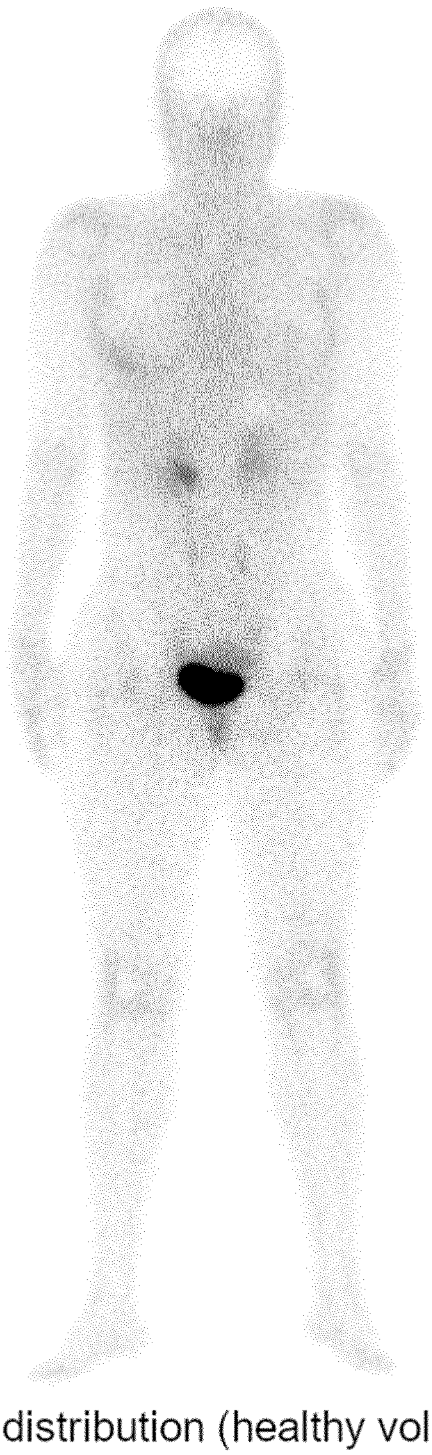

Normal distribution (healthy volunteer)

Figure 3

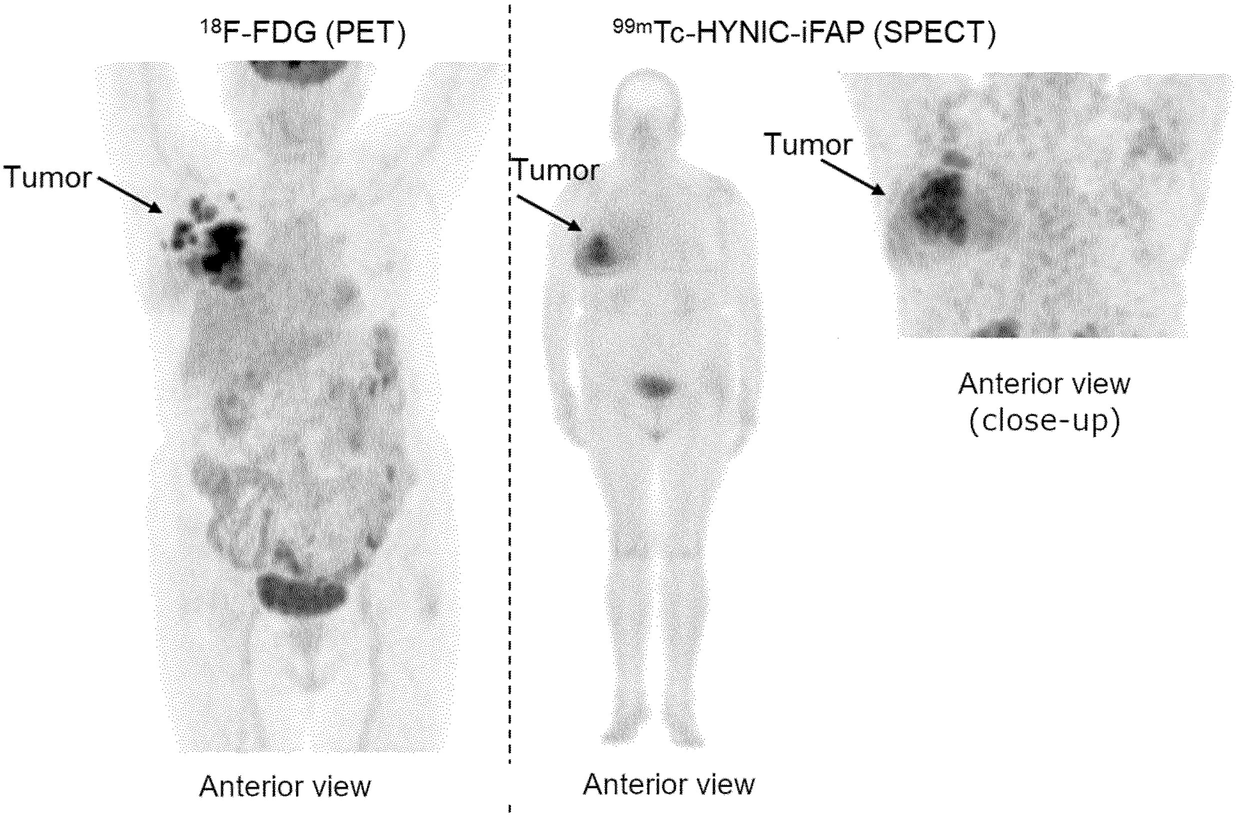

$^{18}$F-FDG (PET)
$^{99m}$Tc-HYNIC-iFAP (SPECT)
Tumor
Tumor
Tumor
Anterior view
Anterior view
Anterior view
(close-up)


**Figure 4**

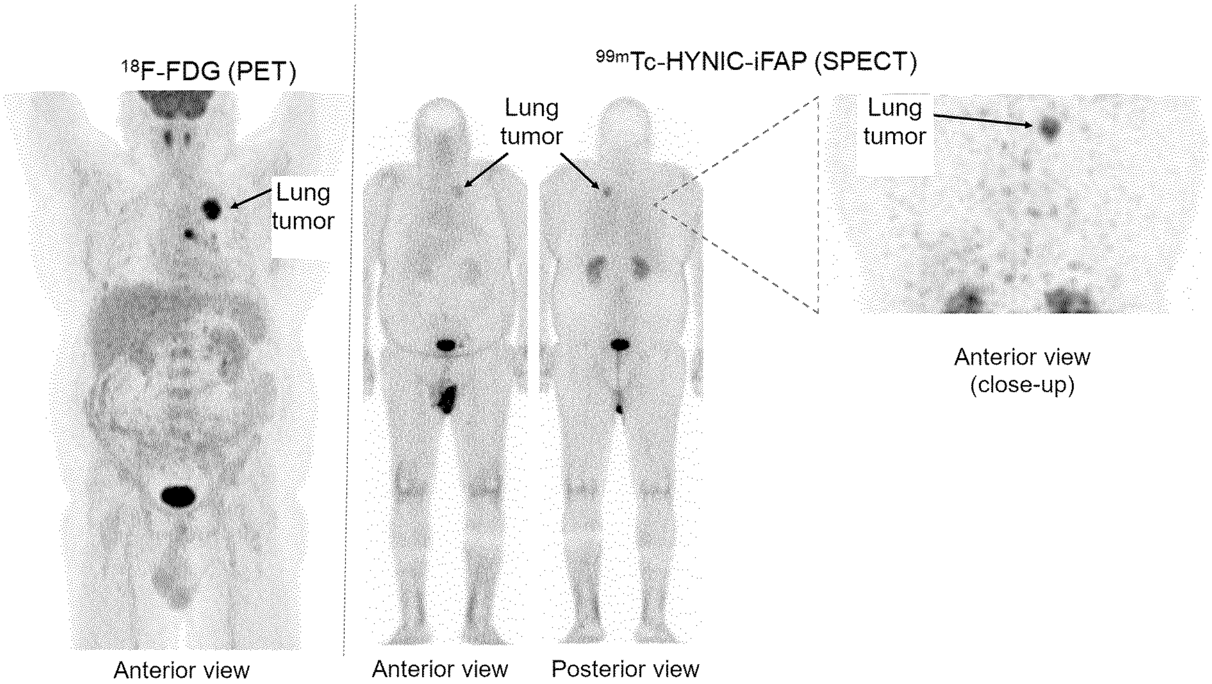
$^{18}$F-FDG (PET)
Lung tumor
Anterior view
$^{99m}$Tc-HYNIC-iFAP (SPECT)
Lung tumor
Lung tumor
Anterior view
Posterior view
Anterior view (close-up)

**Figure 5**

# INTERNATIONAL SEARCH REPORT

International application No.
PCT/MX2021/050055

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
A61K, G01N, C07D, C07F, A61P

Documentation searched other than minimum documentation to the extent that such documents are included in the fields searched

Electronic data base consulted during the international search (name of data base and, where practicable, search terms used)

EPODOC, INVENES, WPI, XPESP, CAPLUS, REGISTRY, NPL

C. DOCUMENTS CONSIDERED TO BE RELEVANT

| Category* | Citation of document, with indication, where appropriate, of the relevant passages | Relevant to claim No. |
|---|---|---|
| Y | POPLAWSKI, S.E. et al. Identification of Selective and Potent Inhibitors of Fibroblast Activation Protein and Prolyl Oligopeptidase. Medicinal Chemistry, 17/04/2013, Vol. 56, pages 3467-3477, <DOI: 10.1021/jm400351a> Abstract, Table 2, Example 8. | 1-4 |
| Y | WO 2019154886 A1 (UNIV HEIDELBERG [DE]) 15/08/2019, Formula (I) page 23, page 57 line 17-page 58 line 3, page 57 lines 1-11, page 4 lines 9-12, page 50 lines 12-17. | 1-4 |
| Y | EP 3486249 A1 (INSTITUTO NAC DE INVESTIGACIONES NUCLEARES [MX]) 22/05/2019, paragraph [0008] and figure 1. | 1-4 |

☒ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| | |
|---|---|
| Date of the actual completion of the international search<br>21/03/2022 | Date of mailing of the international search report<br>**(22/03/2022)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>B. de Luis Fernández<br><br>Telephone No. 91 3495451 |

Form PCT/ISA/210 (second sheet) (January 2015)

## INTERNATIONAL SEARCH REPORT

International application No.
PCT/MX2021/050055

C (continuation). DOCUMENTS CONSIDERED TO BE RELEVANT

| Category * | Citation of documents, with indication, where appropriate, of the relevant passages | Relevant to claim No. |
|---|---|---|
| Y | WO 2017165473 A1 (UNIV JOHNS HOPKINS [US]) 28/09/2017, page 3 lines 11-14, Formula (I) page 5, page 5 lines 9-12, page 6 lines 4-9, Example P1 page 7, page 30 lines 10-13. | 1-4 |
| P,X | WO 2021195198 A1 (TUFTS COLLEGE [US]) 30/09/2021, page 2 lines 10-12, Formula II page 4, page 7 lines 7-11, page 22 lines 8-12, example 5183 page 52, example 5180 page 74, example 3860 page 161. | 1-4 |

Form PCT/ISA/210 (continuation of second sheet) (January 2015)

## INTERNATIONAL SEARCH REPORT

Information on patent family members

International application No.
PCT/MX2021/050055

| Patent document cited in the search report | Publication date | Patent family member(s) | Publication date |
|---|---|---|---|
| WO2019154886 A1 | 15.08.2019 | RU2020126278 A | 09.03.2022 |
| | | RU2020126278 A3 | 09.03.2022 |
| | | JP2021512949 A | 20.05.2021 |
| | | US2021038749 A1 | 11.02.2021 |
| | | BR112020015985 A2 | 15.12.2020 |
| | | CL2020002026 A1 | 27.11.2020 |
| | | KR20200123148 A | 28.10.2020 |
| | | CN111699181 A | 22.09.2020 |
| | | SG11202007180Q A | 28.08.2020 |
| | | CO2020009625 A2 | 21.08.2020 |
| | | CA3088326 A1 | 15.08.2019 |
| | | AU2019219057 A1 | 09.07.2020 |
| | | EP3749663 A1 | 16.12.2020 |
| | | WO2019154859 A1 | 15.08.2019 |
| EP3486249 A1 | 22.05.2019 | PT3486249T T | 02.02.2022 |
| | | DK3486249T T3 | 07.02.2022 |
| | | MA45687 A | 31.03.2021 |
| | | US2021187132 A1 | 24.06.2021 |
| | | ZA201807828 B | 31.03.2021 |
| | | US2019343970 A1 | 14.11.2019 |
| | | US10918745 B2 | 16.02.2021 |
| | | CA3059545 A1 | 28.12.2017 |
| | | CA3059545 C | 27.07.2021 |
| | | EA201892668 A1 | 31.10.2019 |
| | | BR112018076593 A2 | 16.04.2019 |
| | | CN109641924 A | 16.04.2019 |
| | | CN109641924B B | 25.02.2022 |
| | | MX2016008466 A | 25.12.2017 |
| | | WO2017222362 A1 | 28.12.2017 |
| WO2017165473 A1 | 28.09.2017 | PL3433238T T3 | 13.12.2021 |
| | | HUE055607T T2 | 28.12.2021 |
| | | HRP20211386T T1 | 10.12.2021 |
| | | SI3433238T T1 | 30.11.2021 |
| | | ES2877572T T3 | 17.11.2021 |
| | | RS62274 B1 | 30.09.2021 |
| | | LT3433238T T | 27.09.2021 |
| | | DK3433238T T3 | 06.09.2021 |
| | | PT3433238T T | 28.07.2021 |
| | | RU2021115958 A | 12.07.2021 |
| | | EP3925952 A1 | 22.12.2021 |
| | | EP3925952 A4 | 22.12.2021 |
| | | US2020155713 A1 | 21.05.2020 |
| | | RU2018133693 A | 22.04.2020 |
| | | RU2018133693 A3 | 22.04.2020 |
| | | JP2019519467 A | 11.07.2019 |
| | | PH12018502048 A1 | 01.07.2019 |
| | | TR201813644T T1 | 21.11.2018 |
| | | CL2018002683 A1 | 22.04.2019 |

Form PCT/ISA/210 (patent family annex) (January 2015)

## INTERNATIONAL SEARCH REPORT

Information on patent family members

International application No.
PCT/MX2021/050055

| Patent document cited in the search report | Publication date | Patent family member(s) | Publication date |
|---|---|---|---|
| | | MX2018011519 A | 28.01.2019 |
| | | BR112018069507 A2 | 29.01.2019 |
| | | CN109311827 A | 05.02.2019 |
| | | KR20180134918 A | 19.12.2018 |
| | | AU2017238181 A1 | 18.10.2018 |
| | | AU2017238181B B2 | 27.05.2021 |
| | | CA3018709 A1 | 28.09.2017 |
| | | EP3433238 A1 | 30.01.2019 |
| | | EP3433238 A4 | 11.09.2019 |
| WO2021195198 A1 | 30.09.2021 | NONE | |

Form PCT/ISA/210 (patent family annex) (January 2015)

## INTERNATIONAL SEARCH REPORT

International application No.
PCT/MX2021/050055

CLASSIFICATION OF SUBJECT MATTER

***A61K51/04*** (2006.01)
***A61K31/69*** (2006.01)
***G01N33/60*** (2006.01)
***G01N33/574*** (2006.01)
***C07D401/12*** (2006.01)
***C07F13/00*** (2006.01)
***C07F5/02*** (2006.01)
***A61P35/00*** (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HAMSON et al.** Understanding fibroblast activation protein (FAP): substrates, activities, expression and targeting for cancer therapy. *Proteomics Clin. Appl.,* 2014, vol. 8, 454-463
- **AERTGEERTS et al.** Structural and kinetic analysis of the substrate specificity of human fibroblast activation protein a. *J. Biol. Chem.,* 2005, vol. 280, 19441-19444
- **EDOSADA et al.** Selective inhibition of fibroblast activation protein protease based on dipeptide substrate specificity. *J. Biol. Chem.,* 2006, vol. 281, 7437-7444
- **TRAN et al.** Synthesis and structure-activity relationship of N-acyl-Gly-, N-acyl-Sar- and N-blocked-boroPro inhibitors of FAP, DPP4, and POP. *Bioorg. Med. Chem. Lett.,* 2007, vol. 17, 1438-1442
- **JANSEN et al.** Extended structure activity relationship and pharmacokinetic investigation of (4-quinolinoyl)-glycyl-2-cyanopyrrolidin inhibitors of fibroblast activation protein. *J. Med. Chem.,* 2014, vol. 57, 3053-3074
- **JANSEN et al.** Selective inhibitors of fibroblast activation protein (FAP) with a (4-quinolinoyl)-glycyl-2-cyanopyrrolidin scaffold. *ACS Med. Chem. Lett.,* 2013, vol. 4, 491-496
- **POPLAWSKI et al.** Identification of selective and potent inhibitors of fibroblast activation protein and prolyl oligopeptidase. *J. Med. Chem.,* 2013, vol. 56, 3467-3477
- **MELETTA et al.** Evaluation of the radiolabeled boronic acid based FAP inhibitor MIP-1232 for atherosclerotic plaque imaging. *Molecules,* 2015, vol. 20, 2081-2099
- **LINDNER et al.** Development of quinoline-based theranostic ligands for the targeting of fibroblast activation protein. *J. Nucl. Med.,* 2018, vol. 59, 1415-1422
- **LOKTEV et al.** A tumor-imaging method targeting cancer-associated fibroblasts. *J. Nucl. Med.,* 2018, vol. 59, 1423-1429
- **LOKTEV et al.** Development of Fibroblast Activation Protein-Targeted Radiotracers with Improved Tumor Retention. *J. Nucl. Med.,* 2019, vol. 60, 1421-1429
- **GIESEL et al.** FAPI-74 PET/CT Using Either F-AIF or Cold-Kit Ga Labeling: Biodistribution, Radiation Dosimetry, and Tumor Delineation in Lung Cancer Patients. *J. Nucl. Med.,* 2021, vol. 62, 201-207
- **LINDNER et al.** Design and Development of Tc-Labeled FAPI Tracers for SPECT Imaging and Re Therapy. *J. Nucl. Med.,* 2020, vol. 61, 1507-1513
- **GIESEL et al.** Ga-FAPI PET/CT: biodistribution and preliminary dosimetry estimate of 2 DOTA-containing FAP-targeting agents in patients with various cancers. *J. Nucl. Med.,* 2019, vol. 60, 386-392
- **KRATOCHWIL et al.** Ga-FAPI PET/CT: Tracer Uptake in 28 Different Kinds of Cancer. *J. Nucl. Med.,* 2019, vol. 60, 801-805
- **WATABE et al.** Theranostics Targeting Fibroblast Activation Protein in the Tumor Stroma: Cu- and Ac-Labeled FAPI-04 in Pancreatic Cancer Xenograft Mouse Models. *J. Nucl. Med.,* 2020, vol. 61, 563-569
- **PARK et al.** Differential expression of cancer-associated fibroblast-related proteins according to molecular subtype and stromal histology in breast cancer. *Breast. Cancer Res. Treat.,* 2015, vol. 149, 727-741